# EUROPEAN PATENT SPECIFICATION

(11) **EP 1 872 821 B1**
(45) Date of publication and mention of the grant of the patent: **29.10.2008**
(21) Application number: 07020121.5
(22) Date of filing: 27.09.2005
(51) Int. Cl.: A61M 25/09

(54) **A medical guide wire**
Medizinischer Führungsdraht
Fil de guidage médical

(43) Date of publication of application: 02.01.2008
(62) Divisional of application: 05256018.2
(73) Proprietor: ASAHI INTECC CO., LTD., Nagoya-shi, Aichi-ken (JP)
(72) Inventor: Kato, Tomihisa, Nagoya-shi Aichi-ken (JP)
(74) Representative: Price, Nigel John King

(56) References cited:
- EP-A- 0 666 086
- EP-A- 0 803 266
- EP-A- 0 982 046
- EP-A- 1 498 152
- EP-A- 1 576 980
- US-A1- 2004 167 436

## Description

The invention relates to a medical guide wire which is improved at manipulatability so that it can navigate deeply into the vascular system by making an efficient use of the blood streams.

Prior to using a catheter, it is necessary to firstly insert a medical guide wire (referred merely to as "guide wire" hereinafter) into a blood vessel as a guide for the catheter. In order to make the guide wire reachable at desired areas of sinuously curved portions in the vascular system, various contrivances have been suggested.

By way of illustration, Japanese Laid-open Patent Application No. 2000-135289 discloses a guide wire in which a radiopaque helical spring wire is connected to a distal end of an elongation core with the helical spring wire coated by a synthetic tube. On an outer surface of the helical spring wire, a hydrophilic polymer is coated to insure a smoothness and slidability so as to protect the helical spring wire against the thrombi formation. The distal end portion of the elongation core is thinned to insure a good pushability upon inserting it into the blood vessel.

Japanese Laid-open Patent Application No. 4-9162 discloses a guide wire, a distal end portion of which is highly flexible with a main portion maintained highly rigid. Into the distal end portion of the guide wire, an X-ray opaque metal is embedded with the distal end portion coated by a synthetic layer. The synthetic layer exhibits a lubricity to insure a good pushability and retractability upon manipulating the guide wire.

Japanese Utility Model Registration No. 2588582 discloses a guide wire in which an elongation core has a distal end portion connected to a radiopaque helical spring. The elongation core is covered by a synthetic coat and a hydrophilic polymer to insure a good manipulatability with a friction reduced against the blood vessel.

Documents EP 1498152A, EP 1576980A, EP 0982046A, US 2004/167436A1, EP 0803266A and EP 0666086A disclose guide wire tips with hollow chambers.

With these related arts in mind, none of the guide wires has an intention to make an effective use of a buoyance developed in the blood streams in order to insure a good manipulatability upon navigating the guide wire through the blood vessel.

Therefore, it is an object of the invention to make an effective use of a buoyance developed in the blood streams, and provide a medical guide wire which forms a flotage chamber to improve a manipulatability upon steering the guide wire even when the guide wire has a distal end portion liable to hang in the blood streams under the influence of the gravity.

### SUMMARY OF THE INVENTION

According to the invention, there is provided the medical guide wire comprising:
a helical spring having a radiopaque portion defined at least on a distal end portion of said helical spring;
an elongate core having a thinned portion at a distal end portion and having a thickened portion at a proximal end portion of said elongate core, said distal end portion of said elongate core being placed within said helical spring, and an outer surface of said helical spring being entirely covered by a synthetic coat;
characterised by a flotage chamber defined between said elongate core and said helical spring being selected from one or two groups consisting of a cell encapsulated by a foamy body, a cell encapsulated by cotton fibers or a bundle of fibers and a cell encapsulated by foamy beads or microballoons.

According to an aspect of the invention, a foamy body may be placed in the flotage chamber between the helical spring and the elongation core.

With the foamy body encapsulated into the flotage chamber, it is possible to prevent the elongation core and the helical spring from being unfavorably deformed so as to increase an elastic restitution after manipulatively bent.

According to an aspect of the invention, cotton fibers or a bundle of fibers may be placed in the flotage chamber between the helical spring and the elongation core.

With the cotton fibers or the bundle of fibers easily adjustable in its quantity, it is possible to readily form the flotage chamber without hindering the flexibility required for the distal end portion of the guide wire.

According to an aspect of the invention, foamy beads or microballoons may be placed in the flotage chamber between the helical spring and the elongation core.

With the foamy beads or microballoons having less chances to come in contact with the neighboring beads or microballoons, it is possible to insure larger spatial portions favorable to provide the flotage chamber. With the use of inorganic microballoons, it is possible to increase a contractile strength for the distal end portion of the guide wire exhibited when manipulatively bent without leaking a gaseous component out of the flotage chamber.

With the flotage chamber formed by the cotton fibers, the bundle of the fibers, the foamy beads or the microballoons, it is possible to readily form the flotage chamber, while at the same time, increasing the contractile strength for the distal end portion of the guide wire as mentioned above.

According to another aspect of the invention, an outer diameter of the medical guide wire is 0.2541 mm (0.01 inches) and the medical guide wire is adaptd to be inserted into the guiding catheter, an inner diameter of which ranges from 1.7 mm (5F) to 2.0 mm (6F).

With the buoyance used to float the flotage chamber in the blood streams, it is possible to navigate the distal end portion of an elongation core deep into the blood vessel. With the thinned elongation core, it is possible to thin a catheter so as to render it less intrusive against the diseased area, thus mitigating the burden the patient owes.
Fig. 1 is a longitudinal cross sectional view of a medical guide wire according to a first embodiment of the invention;
Fig. 2 is a latitudinal cross sectional view taken along the line I-I of Fig. 1;
Fig. 3 is a plan view of an elongation core;
Fig. 4 is a side elevational view of the elongation core;
Fig. 5 is an explanatory view of the elongation bar, a distal end portion of which is formed into a multi-stepped configuration;
Fig. 6 is an enlarged longitudinal cross sectional view of a medical guide wire according to a second embodiment of the invention;
Fig. 7 is a latitudinal cross sectional view taken along the line III-III of Fig. 6;
Fig. 8 is a latitudinal cross sectional view taken along the line III'-III' of Fig. 6;
Fig. 9 is an enlarged longitudinal cross sectional view of the medical guide wire;
Fig. 10 is a side elevational view of a medical guide wire according to a third embodiment of the invention but partly sectioned;
Fig. 11 is a longitudinal cross sectional view of a main part of a medical guide wire according to a fourth embodiment of the invention;
Fig. 12 is a longitudinal cross sectional view of a main part of a medical guide wire according to a fifth embodiment of the invention;
Fig. 13 is a longitudinal cross sectional view of a main part of a medical guide wire according to a sixth embodiment of the invention;
Fig. 14 is a longitudinal cross sectional view of a main part of a medical guide wire according to a seventh embodiment of the invention; and
Fig. 15 is a longitudinal cross sectional view of a main part of a medical guide wire according to an eighth embodiment of the invention.

In the following description of the depicted embodiments, the same reference numerals are used for features of the same type.

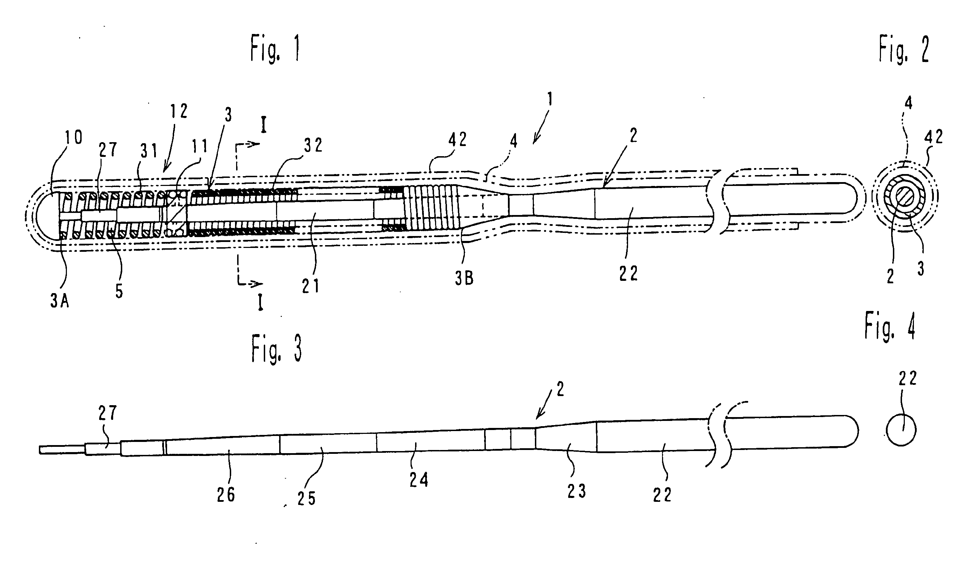

Referring to Figs. 1 through 4 which show a medical guide wire 1 according to a first embodiment of the invention, the medical guide wire 1 (referred only to as "guide wire 1" hereinafter) has an elongation core 2 and a helical spring 3 inserted to an outer surface of a distal end portion 21 of the elongation core 2. The elongation core 2 is formed by a stainless steel wire, and having the distal end portion 21 extended by approx. 300 mm in length with the rest of the guide wire 1 as a proximal end portion 22 extending by approx. 1200 mm or 2700 mm in length.

The distal end portion 21 has an acutely tapered portion 23, a moderately tapered portion 24, a columnar portion 25, a moderately tapered portion 26 and a multi-stepped flat portion 27 (e.g., 0.04 mm, 0.05 mm and 0.063 mm in thickness from the distal end to the proximal end portion).

In this instance, the distal end portion of the elongation core 2 pressingly formed into a multi-stepped flat configuration has such a structure that the distal end portion 21 is pressed with its latitudinal cross section uniformly maintained through its lengthwise direction. The pressing procedure deform the distal end portion 21 steady so as to render the minute dimensions of the multi-stepped flat portion 27 stable, while at the same time, lengthening the service life of a mould die.

Upon forming the helical spring 3, a platinum wire and a stainless steel wire are prepared which are connectedly welded each other, and extruded to be diametrically reduced before helically wound. The helical spring 3 measures approx. 300 mm, a length of which is substantially the same as the distal end portion 12 of the elongation core 2. The helical spring 3 forms a front helical spring tube 31 and a rear helical spring tube 32. The front helical spring tube 31 serves as a radiopaque portion (approx. 50 mm in length), and the rear helical spring tube 32 serves as a radiotransparent portion (approx. 250 mm in length).

A distal end of the helical spring 3 is air-tightly secured to a distal end of the elongation core 2 by means of a brazing procedure, and a proximal end of the helical spring 3 is air-tightly secured to a proximal end of the elongation core 2 by means of a brazing procedure.

On the outer surface of the helical spring 3 and a proximal end portion 22 of the elongation core 2, are covered by a synthetic coat 4 such as urethane layer or the like. On an outer surface of the synthetic coat 4, is covered by a viscous fluid layer 42 (e.g., polyvinylpyrrolidone selected among the hydrophilic polymer).

At a conjunction between the front helical spring tube 31 and the rear helical spring tube 32, there is provided a hermetical wall 11 by means of a brazing procedure. Within the front helical spring tube 31, a flotage chamber 5 is formed as an inner space surrounded by a brazed portion 10, the hermetical wall 11 and the synthetic coat 4. The structure is such that the flotage chamber 5 is placed at a distal portion 12 of the guide wire 1.

The floatge chamber 5 works as follows:
(a) The platinum metal (21.4 in terms of density relative to water employed to the front helical spring tube 31 is approx. 2.7 times as heavy as the stainless steel (7.9 in terms of density relative to water).
(b) Since the distal portion 12 of the guide wire 1 requires a flexibility, the elongation core 2 is thinned. For this reason, the guide wire 1 has the distal portion 12 liable to hang under the influence of gravity as the front helical spring tube 31 becomes heavy. This holds true when the distal portion 12 of the guide wire 1 navigates in the blood streams when inserted into the blood vessel.
(c) Upon inserting the guide wire 1 into the blood vessel, the distal portion 12 of the guide wire 1 generally hangs along the vascular wall, thus increasing a contact area with the vascular wall so as to invite a vascular rupture and a media rupture. Especially at bifurcated portions of the blood vessel, it becomes difficult to selectively manipulate which way to insert the distal portion 12 of the guide wire 1 at the bifurcated portions of the blood vessel.
(d) With the flotage chamber 5 provided in the distal portion 12 of the guide wire 1, it is possible to mitigate the distal portion 12 from hanging in the blood streams, thus making it possible to ride the distal portion 12 on the blood streams to smoothly navigate it deep into the sinuous and meandered blood vessel.
(e) With the flotage chamber 5 air-tightly sealed, it is possible to maintain a good elasticity of the flotage chamber 5 so as to maintain a good restitutive force appeared after manipulatively bending the distal portion 12 of the guide wire 1.
   As a radiopaque material for the front helical spring tube 31, a metal such as gold, silver, tungsten may be selected. As a radiotransparent material for the rear helical spring tube 32, a stainless steel may be preferably selected because of its good biocompatibility.
   The radiotransparent material such as a platinum wire is liable to deform easily with a small amount of springback as compared to a stainless steel wire. Upon winding a linear wire (0.072 mm in diameter) into a helical spring (0.355 mm in outer diameter), it was found that the helical spring (made of the platinum wire) deforms diametrically smaller than the helical spring (made of the stainless steel wire) by 0.02 mm or more. Because the front helical spring tube 31 deforms easily, it is possible to provide a bending tendency with the front helical spring tube 31, and navigate the distal portion 12 of the guide wire 1 staunchly along the sinuous and meandered path upon inserting it deep into the blood vessel.
(f) With the flotage chamber 5 air-tightly sealed in the distal portion 12 of the guide wire 1, the deformation of the flotage chamber 5 increases its inside pressure, and an increased pressure tends to restitute the flotage chamber 5 after released from the deformation.
   With the use of the elastic restitution of the flotage chamber 5, it is possible to add an tendency to the distal portion 12 to favorably hold its initial configuration.
   Due to the difference of springback between the front helical spring tube 31 and the rear helical spring tube 32, it is possible to diametrically reduce the distal portion 12 of the guide wire 1 progressively as approaching forward.
   The flotage chamber 5 effectuates a shape-holding advantage for the distal portion 12 of the guide wire 1 to significantly improve its passage against the stenotic area of the blood vessel.
(g) With the distal portion 12 riding on the blood streams to navigate deep into a somatic body, it becomes possible to thin the distal portion 12 of the guide wire, thus making it less intrusive to mitigate the burden the patient owes.

By way of illustration, upon implementing the therapeutic dilatation against the cardiovascular stenosis area, i.e., percutaneous transluminal coronary angioplasty (PTCA), a guide wire (0..35 mm in outer diameter) and a catheter (7F-8F: 2.3-2.7 mm in inner diameter) are used to introduce a balloon to dilate the cardiovascular stenosis area. The guide wire used for the therapeutical manipulation is generally 0.355 mm in outer diameter.

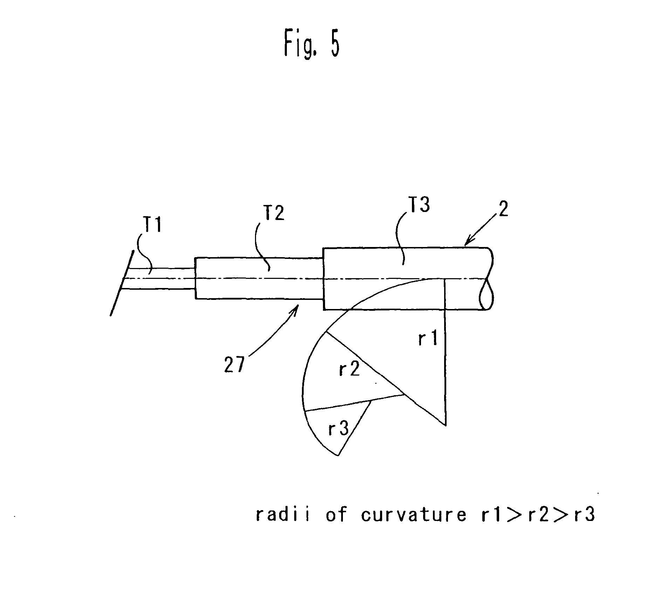

Due to the multi-stepped flat portion 27, as shown in Fig. 5, it is possible to bend stepped sections T1, T2 and T3 of the multi-stepped flat portion 27 at different radii of curvature r1, r2 and r3, thus enabling the manipulator to readily bend the distal end portion 21 of the elongation core 2 and insert it deep into a stenotic area staunchly along a sinuously formed path in the blood vessel.

Because of a buoyance derived from the flotage chamber 5, it is possible to make the distal end portion 21 tide on the blood streams to make it insert deep into the blood vessel. For this reason, the buoyance mitigates the mechanical requirements (e.g., torque transmissibility) for the guide wire 1 to permit the elongation core 2 to be thinned.

By way of illustration, the buoyance enables manufacturers to reduce the guide wire 1 from 0.014 to 0.010 inches (0,356 to 0,254 mm) in outer diameter, while at the same time, thinning a catheter from 7F-8F (2.3-2.7 mm in inner diameter) to 5F-6F (1.7-2.0 mm in inner diameter).

With the helical spring 3 formed from the front helical spring tube 31 and the rear helical spring tube 32, and the flotage chamber 5 provided in the front helical spring tube 31, it is possible to ameliorate an entire balance and prevents the distal portion 12 from being hung heavily.

In the guide wire 1 used for the percutaneous transluminal coronary angioplasty (PTCA), the elongation core 2 is tapered from the rear helical spring tube 32 to a distal end of the front helical spring tube 31. Since the specific gravity of the front helical spring tube 31 is greater with the distal end portion 21 thinned, the guide wire 1 has the distal portion 12 liable to hang as approaching forward.

With the flotage chamber 5 provided in the distal portion 12 of the guide wire 1, it is possible to effectively mitigate the distal portion 12 from being hung in the blood streams, thus making it possible to substantially maintain it straight so as to avoid a vascular rupture and a media rupture in the blood vessel.

The flotage chamber 5 is air-tightly sealed positively by the brazing portion 10, the hermetical wall 11 and the synthetic coat 4. The distal end portions of the elongation core 2 and the helical spring 3 are tightly fixed by means of a brazing procedure (tin pellets), brazing procedure or the like. A proximal end of the front helical spring tube 31 is tightly fixed to the elongation core 2 by means of a brazing procedure (tin pellets), brazing procedure or the like.

Thereafter, the synthetic coat 4 are applied to an outer surface of the front helical spring tube 31 to at least cover an entire surface of the flotage chamber 5.

It is to be noted that the synthetic coat 4 may be applied to a whole part of the helical spring 3 and the elongation core 2. As for a method of forming the synthetic coat 4, an extrusion procedure, a dipping procedure or a thermal shrinkage tube procedure may be used so long as the method is effective in air-tightly sealing the flotage chamber 5.

Among the methods raised above, the dipping procedure and the thermal shrinkage tube procedure are preferable since they prevents the synthetic coat 4 from invading into the flotage chamber 5 with the gaseous component left intact inside the flotage chamber 5. The dipping procedure is also preferable since it neither needs to pressurize the flotage chamber 5 nor needs to thermally deal with the ends of the synthetic coat 4. In order to avoid the gaseous leakage from the flotage chamber 5, double layers of the synthetic coat may be provided.

It is also to be noted that a viscous fluid layer 42 (different in viscosity from the blood streams) may be provided as a hydrophilic polymer on an outermost surface of the double layers. The fluid layuer 42 serves as a second lubricating layer to exhibit a lubricity when moistened.

With the outer surface of the helical spring 3 covered by the synthetic coat 4, it is possible to maintain a good elasticity of the flotage chamber 5 so as to hold a good restitutive force appeared after manipulatively bending the distal portion 12 of the guide wire 1, while at the same time, protecting the elongation core 2 against the plastic deformation.

Due to the double layers forming the viscous fluid layer 42 on the first solid layer (e.g., polyurethane layer) of the synthetic coat 4, the following advantages are obtained.

Even if minute pores (pinholes) or injuries are developed on the synthetic coat 4., it is possible to avoid the gaseous leakage of the flotage chamber 5 so as to air-tightly maintain the flotage chamber 5 by covering an entire surface of the synthetic coat 4 with the viscous fluid layer 42.

By covering the synthetic coat 4 with the viscous fluid layer 42, it is possible to mitigate the friction of the synthetic coat 4 against the vascular wall in the blood vessel.

Upon forming the synthetic coat 4 from a mixture of a hydrophilic polymer and a hydrophobic polymer, it is possible to provide the synthetic coat 4 with a first layer which contains the hydrophobic polymer more than the hydrophilic polymer by weight, and having an outer layer which contains the hydrophilic polymer more than the first layer contains the hydrophilic polymer by weight. The hydrophilic polymer of the outer layer increases progressively by weight as approaching an outer surface of the outer layer. This makes it possible to more air-tightly seal the flotage chamber 5, while and at the same time, mitigating the friction of the synthetic coat 4 against the vascular wall in the blood vessel.

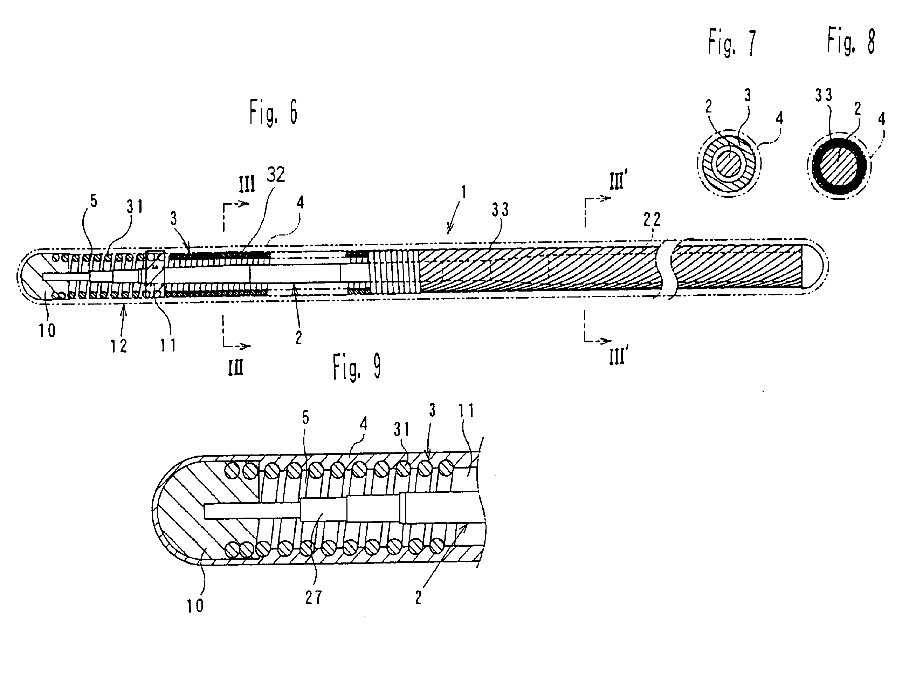

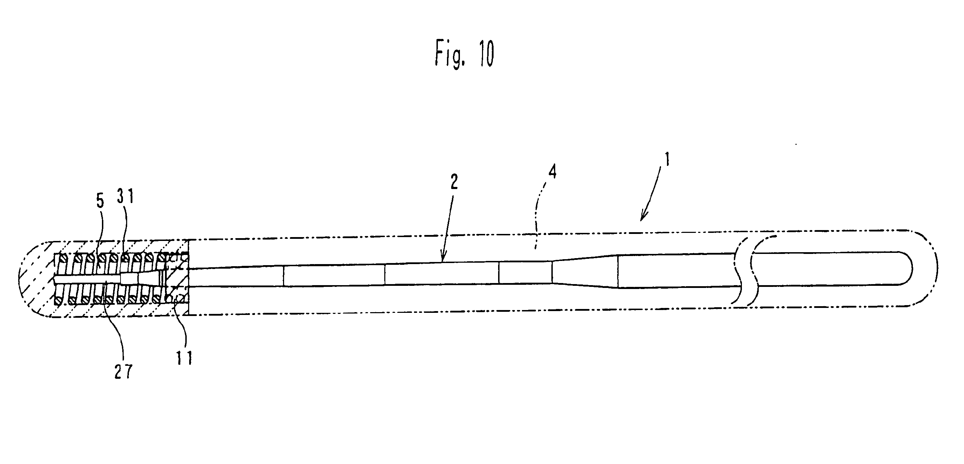

Figs. 7 through 10 show a second embodiment of the invention in which a multi-stranded helical spring tube 33 is connected to a proximal side of the rear helical spring tube 32. Both rear ends of the elongation core 2 and the multi-stranded helical spring tube 33 are fixed by means of a welding or brazing procedure.

When comparing a solid core to the case in which the solid core is inserted to the multi-stranded helical spring under the common diametrical dimension at the proximal end portion, the multi-stranded helical spring tube develops a concave-shaped clearance between the neighboring helical line elements. This makes it possible to produce the lightweight elongation core as opposed to a solid elongation core.

Upon inserting the elongation core 2 into the blood vessel, the blood streams run along the helical line elements of the multi-stranded helical spring tube 33 to give the elongation core 2 a propelling force, thus enabling the manipulator to navigate it deep into the stenotic area of the blood vessel.

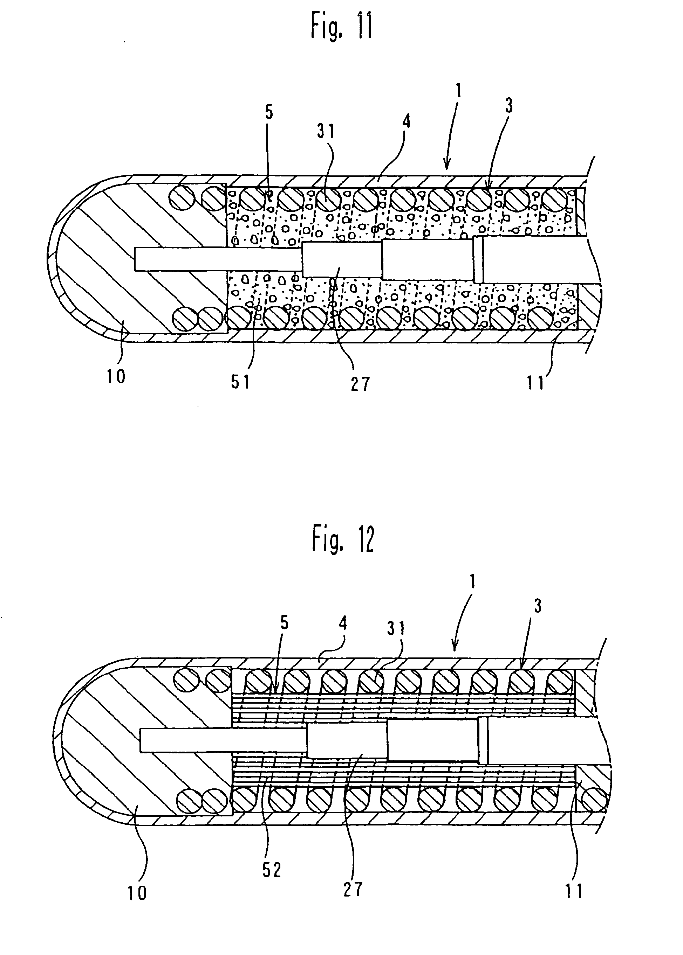

Fig. 11 shows a third embodiment of the invention in which the front helical spring tube 31 forms all the helical spring 3. In this instance, an entire surface of the front helical spring tube 31 is covered by the synthetic coat 4 to provide the guide wire 1.

Fig. 12 shows a fourth embodiment of the invention in which the flotage chamber 5 is formed by filling a foamy substance (sponge) 51 between the helical spring 3 and the elongation core 2. In this instance, the foamy substance 51 is provided by dipping the distal end portion 21 of the elongation core 2 into a foamy liquid bath after brazing the helical spring 3 to the elongation core 2. Then, the elongation core 2 is withdrawn from the foamy liquid bath, and trimmed at the foamy substance 51 to be diametrically constant in the lengthwise direction with the use of a jig tool.

Thereafter, the foamy substance 51 is heated or left as it is until solidified. With the use of the dipping procedure, the synthetic coat 4 is covered with an entire surface of the helical spring 3 and the elongation core 2. It is to be noted that a spray may be used upon providing the foamy substance 51.

The foamy substance 51 is provided by adding a foamy agent to a synthetic resin. The synthetic resin represents polyester, copolymer of styrene and methacrylic acid (styrene-based resin) and polyethylene, polypropylene (polyolefin-based resin). The foaming agent represents carbon dioxide (volatile) and ammonium carbonate (dissoluble). By way of example, a bridged bond polyolefin foaming agent density relative to water : 0.06-0.3 may be used.

The foamy substance 51 may be formed by adding the foamy agent to a rubber (silicone rubber, chloroprene rubber). The foamy agent for the silicone rubber represents azobisisobutylnitrile. As for the foamy substance 51, a texture in which foams are discretely arranged is preferable to a texture in which foams are continuously arranged. The minute foams contained in the foamy substance 51 are preferable. A silicone sponge (minute cell-texture and 110 µm on average cellular diameter) is preferable which has a low density relative to water (approx. 0.41) with an excellent permanent strain exhibited when a contractile stress is applied.

With the foamy substance 51 placed between the elongation core 2 and the helical spring 3, it is possible to preven the synthetic from invading into the flotage chamber 5 even if the helical spring 3 is depressed upon applying the synthetic coat 4 to the outer surface of the helical spring 3 by means of an extrusion procedure.

With the foamy substance 51 formed by the discretely arranged-foam texture, it is possible to effectively avoid the synthetic resin from invading into the flotage chamber 5 upon applying the synthetic coat 4 to the outer surface of the helical spring 3.

Due to the foamy substance 51 being of an elastic material, it effectively prevents the elongation core 2 and the helical spring 3 from being plastically deformed, so as to insure an increased restitutive force appeared after the distal end of the guide wire 1 is manipulatively bent.

In order to provide the flexibility with the front helical spring tube 31, it is arranged to develop a tiny clearance between the helical line elements of the front helical spring tube 31. If the synthetic resin invades into the tiny clearance between the helical line elements upon forming the synthetic coat 4, it would hinder the good flexibility of the front helical spring tube 31.

With the flotage chamber 5 formed by the foamy substance 51, the foamy substance 51 extends over the outer surface of the front helical spring tube 31, thus maintaining the good flexibility of the front helical spring tube 31.

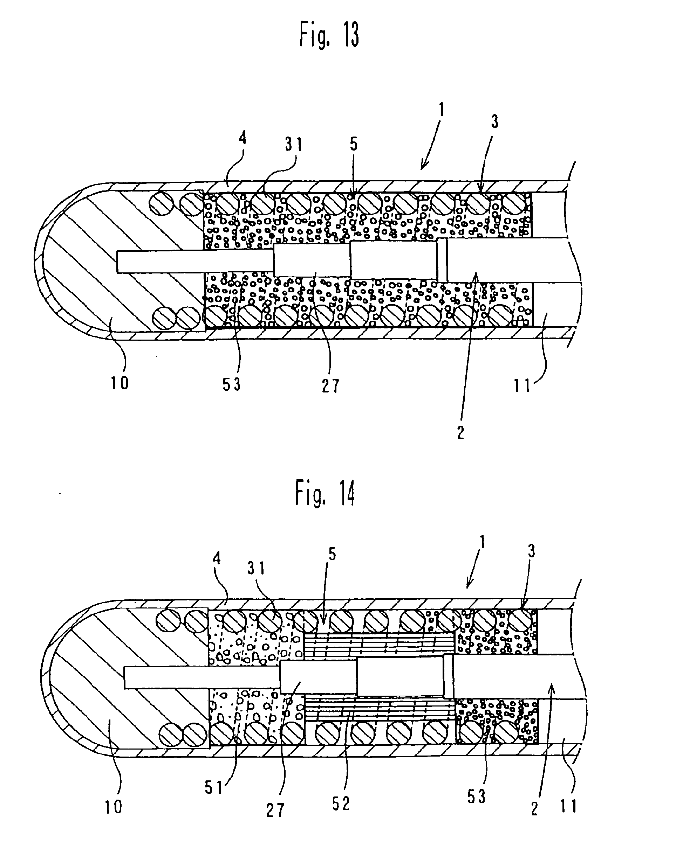

Fig. 13 shows a fifth embodiment of the invention in which the.flotage chamber 5 is formed with cotton fibers or a bundle of fibers 52. They represents polyethylene fibers, para-aramid fibers and PBO fibers. It is preferable that shapes of the fibers (hollow fibers) are such as to contain a gaseous component when the fibers are bundled. The fibers (2-100 µm in thickness) may be bundled in a braid-like configuration. Biocompatible fibers may be used as bioabsorbable polymer fibers (e.g., biodegradable polylactic acid). The fibers measure 0.5-50 µm in diameter and 3-50 mm in length.

With the use of of very thin fibers (2-10 µm), it is possible to form the flotage chamber 5 with the bundle of the fibers 52 which contain a greater amount of the gaseous component. The bundle the fibers 52 favorably maintains the flexibility required for the distal end 12 of the guide wire 1. By appropriately winding the braid around the elongation core 2, it is possible to adjust a wound length of the braid so as to readily form the flotage chamber 5. Upon using the bioabsorbable polymer fibers, it is possible to decompose the fibers within the body, thus involving no complication disorder with no uncomfortable feeling given to the patient even if the fibers flow into the blood streams.

Fig. 13 shows a sixth embodiment of the invention in which the flotage chamber is formed with globular grains 53 (synthetic foamy beads, microballoons). The material for the globular grains 53 (e.g., 0.06-0.5 and 50-100 µm in terms of the density relative to water and granular size) is selected from the chemical components raised in the fourth embodiment of the invention.

As the microballoons, minute and inorganic globular grains (e.g., vitreous, alumina or silica) are selected (e.g., 0.2-0.7 and 1-150 µm in terms of the specific gravity and granular size). The flotage chamber 5 may be formed with a single one substance selected from the synthetic foamy beads and the microballoons. A mixture (binder) of the rubber and the synthetics, the foamy substance 51 or the bundle of fibers 52 may be used upon forming the flotage chamber 5. The globular grains 53 may be formed by the microballoons (e.g., 0.2 and 10 µm in terms of the density relative to water and granular size) mixed with the foamy substance 51.

With the foamy beads or microballoons having less chances to come in contact with the neighboring beads or microballoons, it is possible to insure larger spatial portions favorable to provide the flotage chamber 5. With the use of inorganic microballoons, it is possible to increase the contractile strength for the distal end portion 12 of the guide wire 1 when manipulatively bent without leaking the gaseous component from the flotage chamber 5. It is preferable that lightweight gas (e.g., helium) may be contained in the flotage chamber 5 to increase the buoyance for the flotage chamber 5.

By using the foamy substance 51 as a binder for the globular grains 53, it is possible to readily form the flotage chamber 5 within the front helical spring tube 31, while at the same time, increasing the buoyance by containing the lightweight gas in the flotage chamber 5.

Upon forming the flotage chamber 5, the same method can be used as mentioned in the fourth embodiment of the invention except for the process in which a certain amount of the globular grains 53 is added to the foamy substance 51.

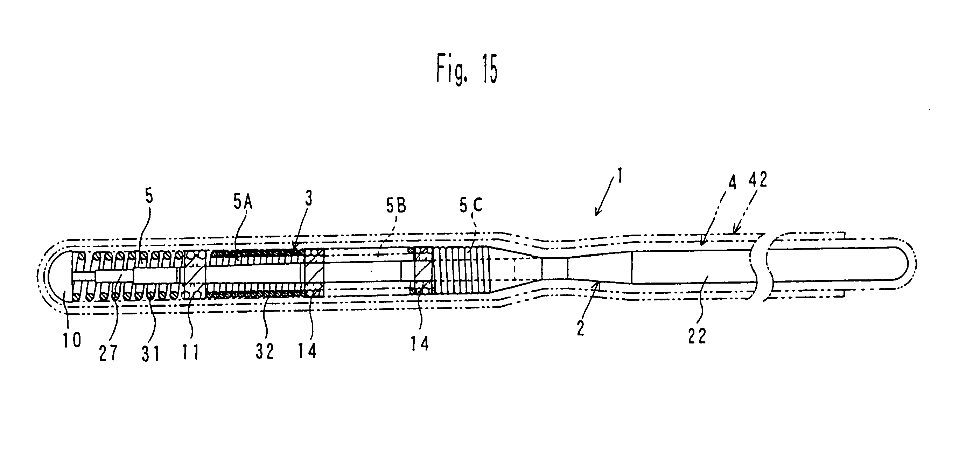

Fig. 15 shows a seventh embodiment of the invention in which the flotage chamber 5 is formed by the globular grains 53, the foamy substance 51, the bundle of fibers 53 or a compound body of these substances. The proximal end of the helical spring 3 may be brazed to the elongation core 2.

In a prior medical guide wire, a radiopaque helical spring is tightly secured to an elongation core by means of a caulking procedure or an adhesive in order to avoid positional displacements between the elongation core and the radiopaque helical spring. This reduces the flexibility required for a distal end of the guide wire.

As opposed to the prior art counterpart, the elongation core 2 and the helical spring 3 are fixed by means of the globular grains 53, the foamy substance 51 or the bundle of fibers 53 in the subject guide wire 1.

For this reason, the helical spring 3 tightly pushes its inner undulating surface against the globular grains 53, thus forming the synthetic coat 4 without inviting the positional displacements between the elongation core 2 and the front helical spring tube 31. The formation of synthetic coat 4 prevents the distal end of the guide wire 1 from losing its flexibility, and effectively avoiding the elongation core 2 and the helical spring 3 from being plastically deformed, so as to insure an increased restitutive force appeared after the distal end of the guide wire 1 is manipulatively bent.

Fig. 16 shows an eighth embodiment of the invention in which a flotage chamber 5A is formed behind the hermetical wall 11 in addition to the flotage chamber 5 provided inside the front helical spring tube 31.

In this instance, hermetical walls 14 are provided inside the rear helical spring tube 32 at regular intervals, and flotage chambers 5A, 5B, 5C are formed inside the rear helical spring tube 32 so as to increase the buoyance for the distal end 12 of the guide wire 1.

The synthetic coat 4 extends from the distal end to the proximal end of the helical spring 3 to air-tightly cover the entire surface of the helical spring 3. The elongation core 2 and the helical spring 3 are fixed by means of not only the brazing procedure but also the plasma welding procedure or the TIG welding procedure so long as the procedures maintain the hermetic seal for the flotage chambers. It is to be noted that the synthetic coat 4 may cover most part of the elongation core 2 as shown in Fig.15.

In this situation, it is possible to make the buoyance increase progressively as approaching forward, while at the same time, making the buoyance adjustable by selecting ones among the flotage chambers 5A, 5B, 5C. This enables the manipulator to favorably direct the distal end 12 of the guide wire 1 along the blood vessel so as to significantly improve a natatorial capability for the guide wire 1.

## Claims

1. The medical guide wire (1) comprising:
a helical spring (3) having a radiopaque portion defined at least on a distal end portion of said helical spring (3);
an elongate core (2) having a thinned portion at a distal end portion and having a thickened portion at a proximal end portion (21) of said elongate core (2), said distal end portion (21) of said elongate core (2) being placed within said helical spring (3), and an outer surface of said helical spring (3) being entirely covered by a synthetic coat (4);
a flotage chamber (5) defined between said elongate core (2) and said helical spring (3) **characterised in that** the flotage chamber (5) being selected from one or two groups consisting of a cell encapsulated by a foamy body (51), a cell encapsulated by cotton fibers or a bundle of fibers and a cell encapsulated by foamy beads or microballoons (53).

2. The combination of a catheter and said medical guide wire (1) according to claim 1, wherein an outer diameter of said medical guide wire (1) is 0.2541 mm (0.01 inches) and said medical guide wire is adapted to be inserted into said catheter, an inner diameter of which ranges from 1.7 mm to 2.0 mm.

## Patentansprüche

1. Medizinischer Führungsdraht (1), umfassend:
eine Schraubenfeder (3) mit einem strahlungsundurchlässigen Abschnitt, der zumindest an einem distalen Endabschnitt der Schraubenfeder (3) ausgebildet ist;
einen langgestreckten Kern (2) mit einem dünner gemachten Abschnitt an einem distalen Endabschnitt und einem dicker gemachten Abschnitt an einem proximalen Endabschnitt (21) des langgestreckten Kerns (2), wobei der distale Endabschnitt (21) des langgestreckten Kerns (2), innerhalb der Schraubenfeder (3) angeordnet ist und eine äußere Oberfläche der Schraubenfeder (3) vollständig von einem synthetischen Überzug (4) bedeckt ist;
eine Schwimmerkammer (5) die zwischen dem langgestreckten Kern (2) und die Schraubenfeder (3) ausgebildet ist,
**dadurch gekennzeichnet, dass**
die Schwimmerkammer ausgewählt ist aus einer oder zwei Gruppen, bestehend aus einer von einem schaumartigen Körper (51) gekapselten Zelle, einer von Baumwollfasern oder einem Bündel von Fasern gekapselten Zelle und einer von schaumartigen Kügelchen oder Mikroballons (53) gekapselten Zelle.

2. Kombination eines Katheters und des medizinischen Führungsdrahts (1) nach Anspruch 1, wobei ein äußerer Durchmesser des medizinischen Führungsdrahts (1) 0,2541 mm (0,01 Zoll) beträgt und der medizinische Führungsdraht geeignet ist in den Katheter eingeführt zu werden, wobei ein innerer Durchmesser desselben sich zwischen 1,7 mm und 2,0 mm bewegt.

## Revendications

1. Fil de guidage médical (1), comprenant :
un ressort hélicoïdal (3) comportant une partie radio-opaque définie au moins sur une partie d'extrémité distale dudit ressort hélicoïdal (3) ;
un coeur allongé (2) comportant une partie amincie à une partie d'extrémité distale et comportant une partie épaissie à une partie d'extrémité proximale (21) dudit coeur allongé (2), ladite partie d'extrémité distale (21) dudit coeur allongé (2) étant disposée à l'intérieur dudit ressort hélicoïdal (3), et une surface extérieure dudit ressort hélicoïdal (3) étant entièrement recouverte par un revêtement synthétique (4) ;
une chambre de flottaison (5) définie entre ledit coeur allongé (2) et ledit ressort hélicoïdal (3),
**caractérisé en ce que** la chambre de flottaison (5) est sélectionnée parmi un ou deux groupes comprenant une cellule encapsulée par un corps en mousse (51), une cellule encapsulée par des fibres de coton ou un faisceau de fibres et une cellule encapsulée par des perles de mousse ou des micro-ballons (53).

2. Combinaison d'un cathéter et dudit fil de guidage médical (1) selon la revendication 1, dans laquelle un diamètre extérieur dudit fil de guidage médical (1) est de 0,2541 mm (0,01 pouce) et ledit fil de guidage médical est adapté pour être inséré dans ledit cathéter, dont un diamètre intérieur est compris entre 1,7 mm et 2,0 mm.
